Europäisches Patentamt
European Patent Office
Office européen des brevets

(19)

(11) Veröffentlichungsnummer: **0 346 841 A2**

(12) # EUROPÄISCHE PATENTANMELDUNG

(21) Anmeldenummer: **89110707.0**

(22) Anmeldetag: **13.06.89**

(51) Int. Cl.4: **C07D 295/18 , A01N 37/38 , A01N 37/18 , C07C 103/76 , C07D 317/50 , A01N 43/30 , C07D 319/18 , A01N 43/32 , C07D 265/30 , C07D 261/02 , A01N 43/80**

Patentanspruch für folgenden Vertragsstaat: ES

(30) Priorität: **16.06.88 CH 2321/88**

(43) Veröffentlichungstag der Anmeldung: **20.12.89 Patentblatt 89/51**

(84) Benannte Vertragsstaaten: **AT BE CH ES FR GB GR IT LI LU NL**

(71) Anmelder: **CIBA-GEIGY AG**
**Klybeckstrasse 141**
**CH-4002 Basel(CH)**

(72) Erfinder: **Riebli, Peter**
**Bünten 17**
**CH-4446 Buckten(CH)**

(74) Vertreter: **Zumstein, Fritz, Dr. et al**
**Bräuhausstrasse 4**
**D-8000 München 2(DE)**

(54) **1-Aryl-naphthoesäureamide und diese enthaltende mikrobizide Mittel.**

(57) 1-Aryl-2-naphthoylamine der Formel I werden beschrieben,

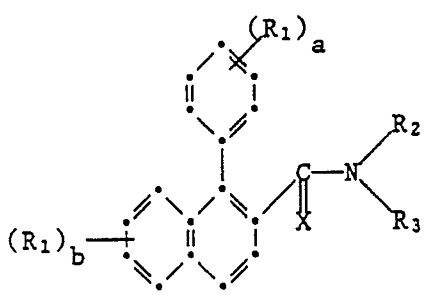

(I)

worin $R_1$, Halogen, Nitro, Cyano, unsubstituiertes oder durch Halogen und/oder $C_1$-$C_3$-Alkoxy ein- oder mehrfach substituiertes $C_1$-$C_6$-Alkyl, $C_3$-$C_6$-Cycloalkyl, $OR_4$, $NR_5R_6$, $CO_2R_5$, $CONR_5R_6$ oder $NHCOR_7$ bedeutet, oder worin zwei benachbarte Positionen im Kern durch eine unsubstituierte oder durch Fluor ein- oder mehrfach substituierte Methylendioxy- oder Aethylendioxy-Gruppe überbrückt wird, worin ferner $R_4$ Wasserstoff oder $C_1$-$C_6$-Alkyl bedeutet, das unsubstituiert oder durch $C_1$-$C_3$-Alkoxy oder ein- oder mehrfach durch Halogen substituiert ist, oder aber für $C_3$-$C_4$-Alkenyl, 2-Propinyl, 3-Halogen-2-propinyl, $-O\underset{O}{\overset{}{C}}ZR_7$ oder $COR_7$ steht,
$R_5$ und $R_6$ unabhängig voneinander H oder $C_1$-$C_4$-Alkyl ist,
$R_7$ für $C_1$-$C_4$-Alkyl und
Z für O oder NH steht,

EP 0 346 841 A2

a und b die Zahl der besetzten Positionen im jeweiligen Ring darstellen und
a für die Zahlen 1-3, b für die Zahlen 0-3 stehen, wobei die einzelnen Gruppen $R_1$ gleich oder verschieden sind, wenn die Summe von a und b grösser als 1 ist,
X für O, S oder NH stehen,
$R_2$ und $R_3$ unabhängig voneinander unsubstituiertes oder durch $C_1$-$C_4$-Alkoxy, Halogen, Cyano substituiertes $C_1$-$C_6$-Alkyl oder aber $C_3$-$C_7$-Cycloalkyl, $C_3$-$C_7$-Alkenyl oder den Rest

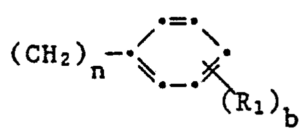

darstellt, worin
n = 0 oder 1 ist, ferner
$R_2$ und $R_3$ auch gemeinsam eine $C_4$-$C_7$-Alkylenkette, welche mit dem sie verbindenden Stickstoffatom einen unsubstituierten oder durch $C_1$-$C_4$-Alkyl mono- oder disubstituierten Heterocyclus bilden kann, wobei eine Methylengruppe aus dieser Kette durch O, S oder $NR_7$ ersetzt sein kann.

Die Verbindungen der Formel I haben mikrobizide Wirkung und lassen sich vorteilhaft für den Pflanzenschutz einsetzen.

## Mikrobizide Mittel

Die vorliegende Erfindung betrifft substituierte 1-Aryl-2-naphthoylamine, deren Herstellung, sowie mikrobizide Mittel, die als Wirkstoff mindestens eine dieser Verbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Die erfindungsgemässen Verbindungen haben die allgemeine Formel I

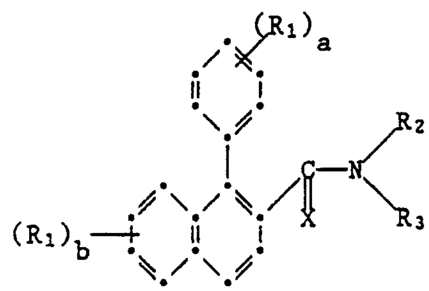

(I)

worin $R_1$ Halogen, Nitro, Cyano, unsubstituiertes oder durch Halogen und/oder $C_1$-$C_3$-Alkoxy ein- oder mehrfach substituiertes $C_1$-$C_6$-Alkyl, $C_3$-$C_6$-Cycloalkyl, $OR_4$, $NR_5R_6$, $CO_2R_5$, $CONR_5R_6$ oder $NHCOR_7$ bedeutet, oder worin zwei benachbarte Positionen im Kern durch eine unsubstituierte oder durch Fluor ein- oder mehrfach substituierte Methylendioxy- oder Aethylendioxy-Gruppe überbrückt sind, worin ferner $R_4$ Wasserstoff oder $C_1$-$C_6$-Alkyl bedeutet, das unsubstituiert oder durch $C_1$-$C_3$-Alkoxy oder ein- oder mehrfach durch Halogen substituiert ist, oder aber für $C_3$-$C_4$-Alkenyl, 2-Propinyl, 3-Halogen-2-propinyl, $-O\underset{\underset{O}{\|}}{C}ZR_7$ oder $COR_7$ steht,

$R_5$ und $R_6$ unabhängig voneinander oder $C_1$-$C_4$-Alkyl ist,

$R_7$ für $C_1$-$C_4$-Alkyl und

Z für O oder NH steht,

a und b die Zahl der besetzten Positionen im jeweiligen Sechsring darstellen, und

a für die Zahlen 1-3 und b für die Zahlen 0-3 steht, wobei die einzelnen Gruppen $R_1$ gleich oder verschieden sind, wenn die Summe von a und b grösser als 1 ist,

X für Sauerstoff, Schwefel oder NH steht,

$R_2$ und $R_3$ unabhängig voneinander unsubstituiertes oder durch $C_1$-$C_4$-Alkoxy, Halogen, Cyano substituiertes $C_1$-$C_6$-Alkyl oder aber $C_3$-$C_7$-Cycloalkyl, $C_3$-$C_7$-Alkenyl oder den Rest

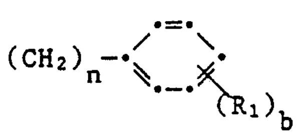

darstellt, worin

n = 0 oder 1 ist, ferner

$R_2$ und $R_3$ auch gemeinsam eine $C_4$-$C_7$-Alkylenkette, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O, S oder $NR_7$ ersetzt sein kann, sowie $R_2$ auch Wasserstoff bedeuten kann; einschliesslich der Additionssalze der Verbindungen der Formel I.

Unter dem Begriff Alkyl selbst oder als Bestandteil eines anderen Substituenten sind je nach der Zahl der angegebenen Kohlenstoffatome beispielsweise folgende Gruppen zu verstehen: Methyl, Ethyl, Propyl, Butyl, Pentyl, Hexyl usw., sowie ihre Isomeren, wie z.B. Isopropyl, Isobutyl, tert.-Butyl, Isopentyl usw.. Cycloalkyl kann beispielsweise Cyclopropyl, Cyclopentyl oder Cyclohexyl bedeuten. Haloalkyl steht für einen einfach bis perhalogenierten Alkylsubstituenten, wie z.B. $CH_2Cl$, $CHCl_2$, $CCl_3$, $CH_2Br$, $CHBr_2$, $CBr_3$, $CH_2F$, $CHF_2$, $CF_3$, $CCl_2F$, $CCl_2$-$CHCl_2$, $CH_2CH_2F$, $CJ_3$ usw.. Unter Halogen soll hier und im folgenden Fluor, Chlor, Brom oder Jod, vorzugsweise Fluor oder Chlor verstanden werden.

Bei Anwesenheit einer Methylendioxygruppe oder Ethylendioxygruppe in einem der Ringe sind damit

zwei benachbarte Positionen besetzt.

Die Verbindungen der Formel I sind unter Normalbedingungen stabile Oele, Harze oder überwiegend kristalline Feststoffe, die sich durch äusserst wertvolle mikrobizide Eigenschaften auszeichnen. Sie lassen sich beispielsweise auf dem Agrarsektor oder verwandten Gebieten präventiv und kurativ zur Bekämpfung pflanzenschädigender Mikroorganismen einsetzen. Die erfindungsgemässen Wirkstoffe der Formel I zeichnen sich in weiten Anwendungskonzentrationen durch eine hohe fungizide Aktivität und problemlose Anwendung aus. Es wird keine Schädigung der behandelten Pflanzen beobachtet.

1-Phenyl-4-hydroxy-2-naphthoylamine der Formel

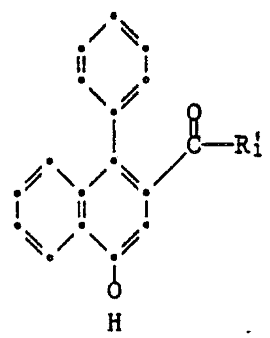

worin $R_1'$ Dimethylamino, Anilino, 4-Chloranilino, 4-Methylanilino oder Benzylamino bedeutet, sind aus der Literatur als Pharmazeutika bekannt (Indian J. Pharm. Sci. 1985 47(1) 12-15; C.A. 103 25, 205882f (1985). Einige dieser Verbindungen wirken entzündungshemmend.

1-Acetoxy- und 1-Hydroxy-3-morpholinocarbonyl-4-phenylnaphthalin der Formel

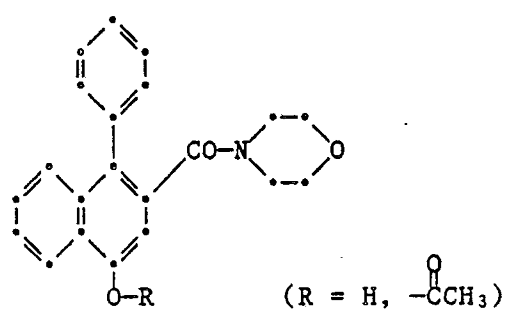

sind im J. Chem. Soc. Perkin Trans. 1 1978 (11), 1360-1366; C.A. 90 (9), 71721e (1979) beschrieben. Gewerblich nutzbare Eigenschaften werden von diesen Verbindungen nicht angegeben.

Aus der Literatur sind keine 1-Aryl-2-naphthoylamine mit mikrobizider Wirkung bekannt.

Ziel der vorliegenden Erfindung ist es, neuartige mikrobizide Wirkstoffe bereitzustellen. Es wurde überraschenderweise gefunden, dass die Verbindungen der Formel I starke mikrobizide Aktivität aufweisen.

Es sind diejenigen Verbindungen der Formel 1 bevorzugt, bei welchen $R_1$ Halogen, Nitro, $C_1$-$C_6$-Alkyl, $C_3$-$C_6$-Cycloalkyl, Methylendioxy, Mono- oder Difluormethylendioxy, unsubstituiertes oder durch Fluor ein- oder mehrfach substituiertes Aethylendioxy, $OR_4$ oder $NR_5R_6$ bedeuten, worin $R_4$ Wasserstoff oder $C_1$-$C_3$-Alkyl darstellt, das unsubstituiert oder durch $C_1$-$C_3$-Alkoxy monosubstituiert oder durch Halogen ein- bis fünffach substituiert ist,
$R_5$ und $R_6$ unabhängig voneinander für H oder $C_1$-$C_4$-Alkyl,
a für die Zahlen 2 oder 3, b für 0 oder 1, und
X für O oder S stehen, während
$R_2$ und $R_3$ unabhängig voneinander Phenyl, 4-Chlorphenyl, ferner $R_2$ und $R_3$ auch gemeinsam eine $C_4$-$C_7$-Alkylenkette bedeuten, welche zusammen mit dem Stickstoffatom einen unsubstituierten oder durch $C_1$-$C_4$-Alkyl mono- oder disubstituierten Heterocyclus bilden können, wobei eine der Methylengruppen aus der Kette durch O, S oder $NR_7$ ersetzt sein kann.

Von dieser Gruppe von Verbindungen sind jene hervorzuheben, bei welchen $R_1$ Methyl, Chlor, Methoxy, Aethoxy, Difluormethoxy, Methylendioxy, Difluormethylendioxy, Aethylendioxy, Nitro, Mono- oder Dimethylamino bedeutet und $R_2$ und $R_3$ mit dem gemeinsamen Stickstoffatom zusammen die Reste

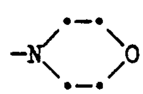

,

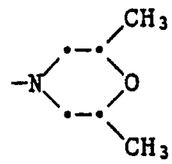

oder

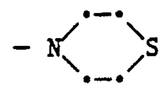

bilden.

Aus dieser Gruppe sind jene Verbindungen von Interesse, bei welchen $R_1$ Methoxy oder Methylendioxy bedeutet und a für die Zahl 2 steht.

Aus der letztgenannten Gruppe stechen jene Verbindungen als Mikrobizide hervor, bei welchen X für Sauerstoff steht.

Als besonders wirksame Einzelverbindung ist aus dieser Gruppe die Verbindung der Formeln Ia anzuführen:

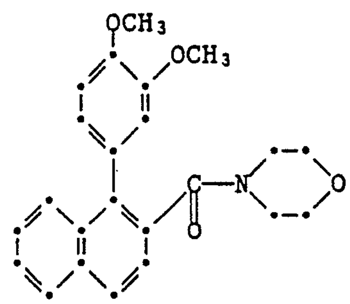

(Ia),

Von den Verbindungen der Formel I, bei welchen $R_1$ Methoxy oder Methyl bedeutet und a für die Zahlen 2 oder 3 und b für O stehen, sind als Mikrobizide jene Verbindungen hervorzuheben, bei welchen X für Schwefel steht.

Als Einzelvertreter ist aus dieser Gruppe die Verbindung der Formel Ib zu nennen:

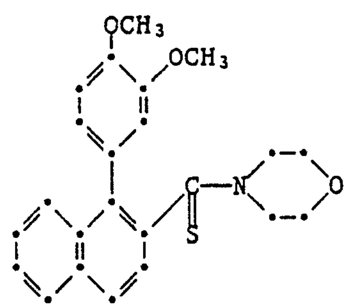

(Ib).

Von den Verbindungen der Formel I, bei welchen $R_1$ Halogen, Nitro, $C_1$-$C_6$-Alkyl, $C_1$-$C_3$-Alkoxy, $C_3$-$C_6$-Cycloalkyl, Methylendioxy, Mono- oder Difluormethylendioxy, unsubstituiertes oder durch Fluor ein- oder mehrfach substituiertes Aethylendioxy oder $NR_5R_6$ bedeuten, worin

$R_5$ und $R_6$ unabhängig voneinander für H oder $C_1$-$C_4$-Alkyl,

a für die Zahlen 2 oder 3 und b für 0 oder 1, und

X für O oder S stehen, und

$R_2$ und $R_3$ unabhängig voneinander für Wasserstoff, unsubstituiertes oder durch $C_1$-$C_4$-Alkoxy, Halogen oder Cyano substituiertes $C_1$-$C_6$-Alkyl, oder oder $C_3$-$C_7$-Alkenyl, sowie Phenyl oder 4-Chlorphenyl stehen, ferner $R_2$ und $R_3$ auch gemeinsam eine $C_4$-$C_7$-Alkylenkette darstellt, welche mit dem Stickstoffatom einen unsubstituierten oder durch $C_1$-$C_4$-Alkyl mono- oder disubstituierten Heterocyclus bilden können, wobei eine der Methylengruppen aus der Kette durch O, S oder $NR_7$ ersetzt sein kann, sind als Mikrobizide jene der Formel Ic bevorzugt,

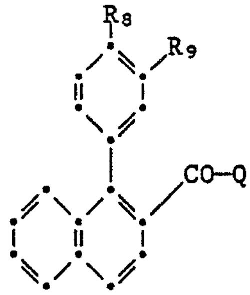

(Ic),

worin $R_8$ und $R_9$ entweder jeweils den Methoxyrest oder zusammen den Methylendioxyrest und Q die folgenden Reste bedeuten:

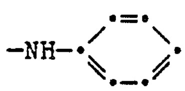 , 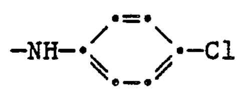 ,

$-N(CH_3)_2$ , $-N(C_2H_5)_2$ , $-N(C_2H_4OCH_3)_2$ ,

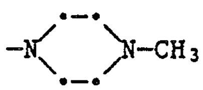 , 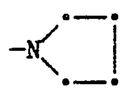 , 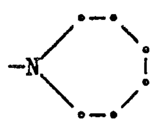 , 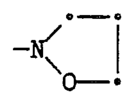 .

Als Einzelvertreter ist aus dieser Gruppe unter anderen die Verbindung der Formel Id zu nennen:

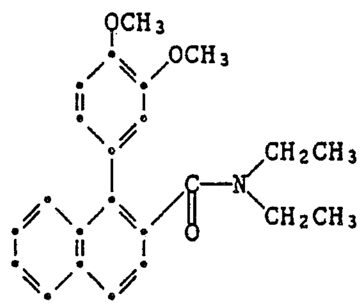

(Id),

Von den Verbindungen der Formel I sind als wertvolle Mikrobizide ausserdem jene der Formel Ie hervorzuheben,

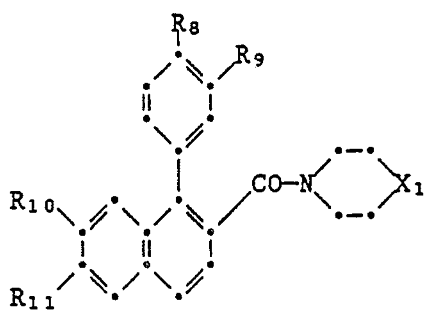

(Ie),

worin $R_8$ und $R_9$ beide entweder Methoxy oder zusammen Methylendioxy bedeuten, und
$R_{10}$ und $R_{11}$ Methoxy oder zusammen Methylendioxy bedeuten und $R_{11}$ ausserdem auch Chlor, Nitro, Amino, Acetylamino sein kann, während
$R_{10}$ Wasserstoff ist, und

$X_1$ O oder S bedeuten.

Bei der erfindungsgemässen Herstellung der Verbindungen der Formel I verfährt man so, dass man ein Amin der Formel II

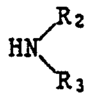 (II),

worin $R_2$ und $R_3$ die für Formel I angegebenen Bedeutungen haben,

a) mit einem Naphthoesäurederivat der Formel III

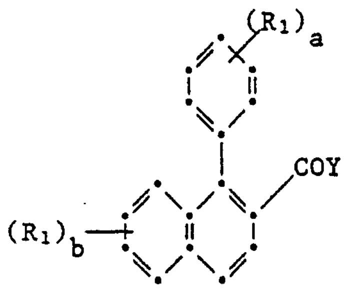 (III),

worin $R_1$, a und b die für Formel I angegebenen Bedeutungen haben und Y OH, Halogen oder $C_1$-$C_4$-Alkoxy bedeutet, in eine Verbindung der Formel I′

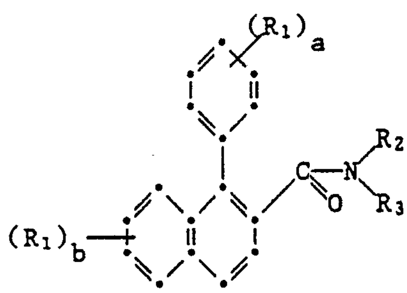 (I')

überführt; und, sofern gewünscht,

die Verbindung I′ mit Phosphorpentasulfid in ein Thiocarbonamid der Formel I″

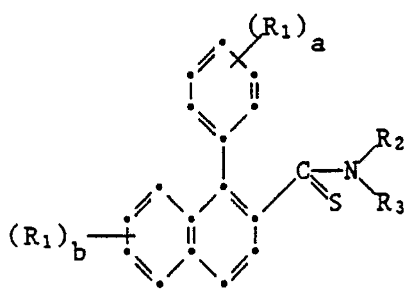 (I'')

verwandelt; oder zur Erzielung von Verbindungen der Formel I mit X = NH,

b) mit einem Iminoester der Formel IV

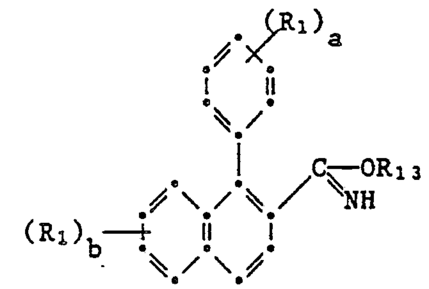

(IV)

worin $R_1$, a und b die für Formel 1 angegebenen Bedeutungen haben und $R_{13}$ $C_1$-$C_4$-Alkyl bedeutet, in eine Verbindung der Formel I‴

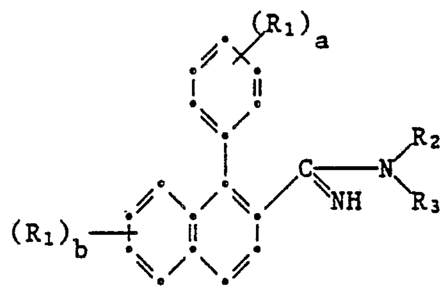

(I''')

überführt.

Bei der Herstellung der Verbindungen der Formel I′ handelt es sich um die Acylierung eines Amins der Formel II mit einem Carbonsäurederivat oder mit einer Carbonsäure der Formel III, wobei letztere vorzugsweise in Gegenwart eines die Säure aktivierenden oder eines wasserentziehenden Mittels eingesetzt wird.

Als säureaktivierende und/oder wasserentziehende Mittel kommen beispielsweise ein Chlorameisensäureester wie Chlorameisensäureäthylester, oder Phosphorpentoxid, N,N′-Dicyclohexylcarbodiimid, N,N′-Carbonyldiimidazol oder N,N′-Thionyldiimidazol in Betracht.

Die Umsetzung wird zweckmässigerweise in einem Lösungsmittel oder Lösungsmittelgemisch wie Methylenchlorid, Chloroform, Tetrachlorkohlenstoff, Aether, Tetrahydrofuran, Dioxan, Benzol, Toluol, Acetonitril oder Dimethylformamid, gegebenenfalls in Gegenwart einer anorganischen Base wie Natriumcarbonat oder einer tertiären organischen Base wie Triethylamin oder Pyridin, welche gleichzeitig als Lösungsmittel dienen kann, und gegebenenfalls in Gegenwart eines säureaktivierenden Mittels bei Temperaturen zwischen -25 °C und 150 °C, vorzugsweise jedoch bei Temperaturen zwischen -10 °C und der Siedetemperatur des Reaktionsgemisches, durchgeführt. Hierbei braucht ein gegebenenfalls im Reaktionsgemisch entstandenes reaktionsfähiges Derivat einer Verbindung der allgemeinen Formeln III nicht-isoliert zu werden, ferner kann die Umsetzung auch in einem Ueberschuss der eingesetzten Verbindung der allgemeinen Formel II als Lösungsmittel durchgeführt werden.

Zur Herstellung solcher Verbindungen der Formel I″ (Thioamide), werden die entsprechenden Amide in einem inerten Lösungsmittel mit Phosphorpentasulfid umgesetzt. In einigen Fällen lässt sich $P_2S_5$ vorteilhaft in Gegenwart von $K_2S$ oder $K_2(S_x)$ einsetzen.

Als Lösungsmittel eignen sich z.B. Toluol, Xylol, Benzol. Die Reaktionstemperatur liegt zwischen 0 °C und der Siedetemperatur des Reaktionsgemischs, wobei jedoch im allgemeinen eine Temperatur von 120 °C nicht überschritten werden sollte.

Zur Herstellung solcher Verbindungen der Formel I‴ (Amidine), werden entsprechende Iminoester mit Aminen der Formel II umgesetzt.

Die Umsetzung erfolgt in einem inerten Lösungsmittel wie Ether oder Tetrahydrofuran bei Temperaturen zwischen etwa 0 und 100 °C.

Carbonsäuren der Formel III sind zum Teil bekannt oder können nach üblichen Verfahren (J. Org. Chem. 1981 46 3881-3886; J. Org. Chem. 1964 29 1757-1762; Synthesis 1983 105-107); hergestellt werden. Iminoester der Formel IV lassen sich aus den entsprechenden Nitrilen durch Anlagerung eines $C_1$-$C_4$-Alkanols in Gegenwart von HCl gewinnen.

Die Amine der Formel II sind allgemein bekannt.

Die Herstellung von Verbindungen der Formel III, worin Y Halogen oder $C_1$-$C_4$-Alkoxy bedeutet kann aus den entsprechenden Carbonsäuren erfolgen.

Die Carbonsäuren der Formel IIIa,

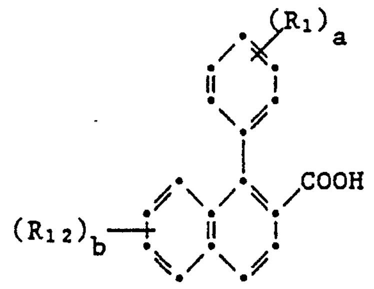

(IIIa),

worin $R_{12}$ Fluor, Chlor oder $C_1$-$C_3$-Alkoxy oder $OR_4$ bedeutet, wobei $R_1$, $R_4$ und auch a und b wie unter Formel I definiert werden, können durch Umsetzung von geeigneten Organometallverbindungen, wie z.B. Grignard-Verbindungen oder Lithiumverbindungen mit Kohlensäure hergestellt werden. Die Organometallverbindungen selbst können durch Metallierung der entsprechenden Bromverbindungen der Formel VIII mit einem geeigneten Metall, wie aktiviertes Magnesium oder mit einer Metallverbindung, wie Buthyllithium nach bekannten Methoden hergestellt werden:

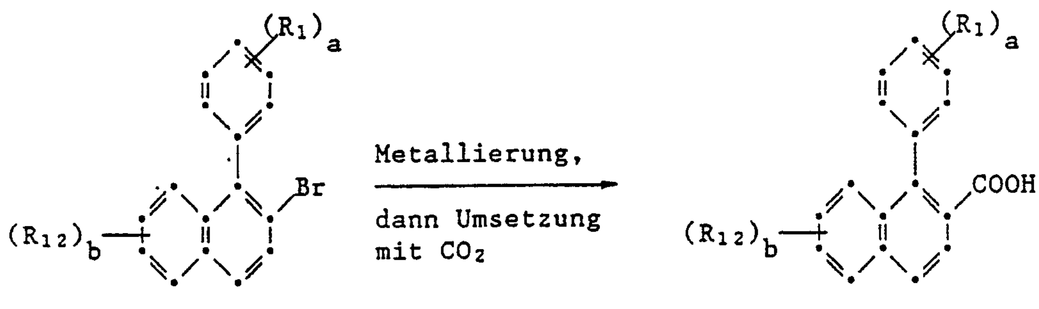


VIII IIIa

Die Bromverbindungen der Formel VIII lassen sich ihrerseits durch Dehydrogenierung der entsprechenden 3,4-Dihydronaphhtaline der Formel VI herstellen. Die Dehydrogenierung kann nach bekannten Methoden, wie z.B. durch Umsetzung mit Dehydrogenierungsmitteln, wie Schwefel oder Chinonen, bevorzugt 2,3-Dichlor-5,6-dicyan-1,4-benzochinon, erfolgen (Chem. Rev. 78 317 1978). Die Verbindungen der Formel VI selbst lassen sich durch Umsetzung von Dihydronaphthalinen der Formel V mit geeigneten Bromierungsmitteln, wie z.B. Pyridiniumhydrobromid-perbromid herstellen (J. Med. Chem. 29 2053-2059, 1986)

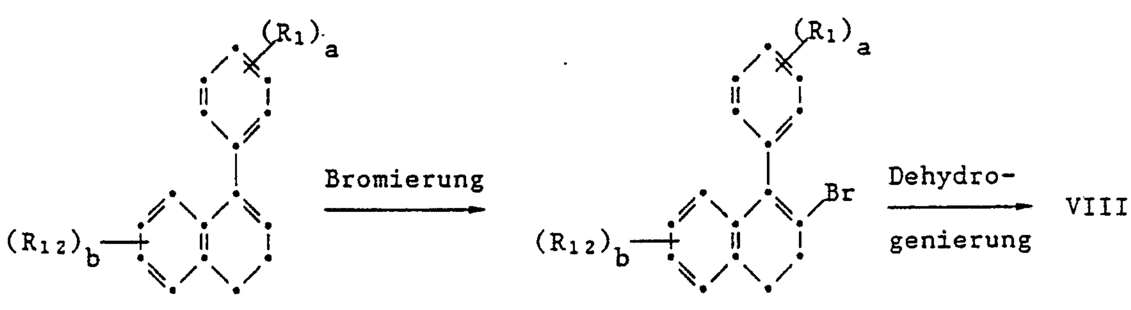


V VI

Durch Variierung der analogen Synthesestufen können die 3,4-Dihydro-Bromnaphthalinverbindungen der Formel VI zunächst über die Metallverbindungen mit Kohlensäure in die 3,4-Dihydronaphthalincarbonsäuren der Formel VII überführt werden, welche dann zum Schluss zu den Verbindungen der Formel IIIa dehydrogeniert werden:

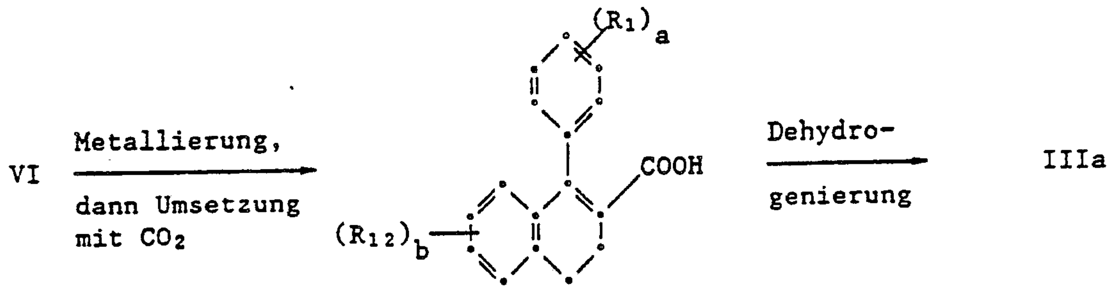


VII

Die Verbindungen der Formel V sind zum Teil bekannt oder können nach bekannten Verfahren [Tetrahedron 38 2403-2410, (1982), Aust. J. Chem. aus 34, 115-129 (1981)] hergestellt werden.

Die Bromnaphthalin-Carbonsäuren der Formel IIIc

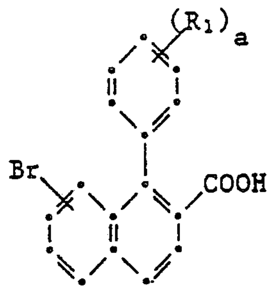

(IIIc),

worin a und $R_1$ wie unter Formel I definiert werden,
können ausgehend aus den entsprechenden nitrierten Naphthalincarbonsäuren der Formel III b nach bekannter Reaktionsfolge (Reduktion, Diazotierung, Sandmeyer-Reaktion) hergestellt werden.

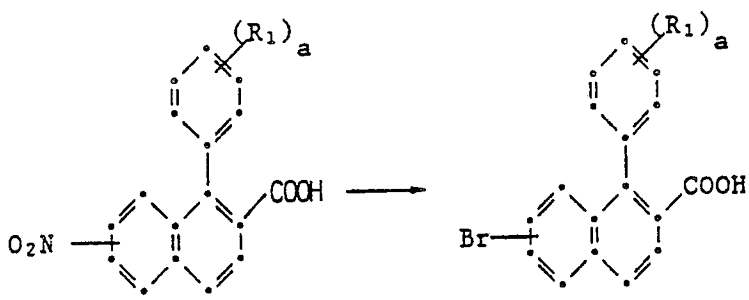

IIIb IIIc

Es wurde nun überraschend festgestellt, dass die erfindungsgemässen Verbindungen der Formel I ein sehr vorteilhaftes, die praktischen Bedürfnisse gut befriedigendes biozides Wirkungsspektrum gegen schädliche Microorganismen, insbesondere gegen phytopathogene Pilze und Bakterien, aufweisen. Sie besitzen sehr vorteilhafte kurative, systemische und insbesondere präventive Eigenschaften und lassen sich zum Schutz von zahlreichen Kulturpflanzen einsetzen. Mit dem Wirkstoff der Formel I können an Pflanzen oder an Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile von phytopathogenen Mikroorganismen verschont bleiben.

Die erfindungsgemässen Wirkstoffe sind beispielsweise gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie insbesondere die der Klasse der Phycomycetes angehörenden Oomycetes wie Phytophthora, Plasmopara, Peronospora, Pythium. Als Pflanzenschutzmittel können die Verbindungen der Formel I auch gegen wichtige Schadpilze aus der Familie der

Fungi imperfecti eingesetzt werden, so z.B. gegen Cercospora, Pyricularia und Botrytis. Ueberdies wirken die Verbindungen systemisch. Darüber hinaus lassen sich Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida. Diese Wirksubstanzen zeigen ferner hervorragende Wirkung gegen Boden- und Samen-bürtige Pilze.

Die Wirkstoffe der Formel I können ferner auch als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden, wobei sie sich insbesondere als Getreidebeizmittel in der Bekämpfung von Pilzorganismen, wie beispielsweise Fusarium-, Helminthosporium und Tilletia-Arten, auszeichnen.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben): Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja): Oelkulturen: (Raps, Senf, Mohn, Oliven, Sonnenblumen, Kokos, Rizinus, Kakao, Erdnüssen); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen-und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin oder Lysolecithin.

Ein bevorzugtes Verfahren zum Aufbringen eines Wirkstoffes der Formel I bzw. eines (agro)chemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln,

Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethylenglykolmonomethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle oder Sojaöl, oder Wasser.

Als feste Trägerstoffe, z.B. für Stäubemittel und dispergierbare Pulver, lassen sich Calcit, Talkum, Kaolin, Montmorillonit oder Attapulgit, hochdisperse Kieselsäure oder saugfähige Polymerisate verwenden. Als gekörnte, adsorptive Granulatträger kommen Bimsstein, Ziegelbruch, Sepiolit oder Bentonit, als nicht als nicht-sorptive Träger z.B. Calcit oder Dolomit in Frage. Es können auch zerkleinerte Pflanzenrückstände verwendet werden.

Als oberflächenaktive Verbindungen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Die in der Formuliertungstechnik gebräuchlichen Tenside sind u.a. in folgenden Publikationen beschrieben:
"Mc Cutcheon's Detergents and Emulsifiers Annual" MC
Publishing Corp., Ridgewood New Jersey, 1980
Sisley and Wood, "Encyclopedia of Surface Active Agents",
Chemical Publishing Co., Inc. New York, 1980.

Die agrochemische Zubereitungen enthalten in der Regel 0,1 bis 99 %, insbesondere 0,1 bis 95 % Wirkstoff der Formel I, 99,9 bis 1 %, insbesondere 99,9 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Derartige (agro)chemische Mittel sind ein Bestandteil der vorliegenden Erfindung.

Die nachfolgenden Beispiele dienen der Illustration der Erfindung, ohne dieselbe einzuschränken (Prozente und Teile beziehen sich auf Gewicht; Temperaturen sind in Celsiusgraden angegeben).

## 1. Herstellungsbeispiele

H.1: Herstellung von 1-(3,4-Dimethoxyphenyl)-2-naphthoesäure-morpholid (Wirksubstanz, Verb. Nr. 1.1, Tabelle 1).

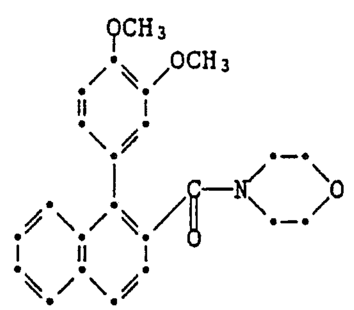

12,3g 1-(3,4-Dimethoxyphenyl)-2-naphthoesäure werden in 70 ml Toluol vorgelegt und mit 4,8 g Thionylchlorid versetzt. Die Reaktionsmischung wird zum Sieden erhitzt und 16 h unter Rückfluss gerührt. Anschliessend werden bei 50° 7,0 g Morpholin zugetropft, das Reaktionsgemisch wird weitere 4h bei Rückfluss gerührt, dann in der Kälte mit 100 ml Toluol verdünnt und 3 x mit je 200 ml Wasser gewaschen. Die organische Phase wird über Natriumsulfat getrocknet, filtriert und das Lösungsmittel unter Vakuum

verdampft. Zur Reinigung wird der ölige Rückstand mittels Ethylacetat über eine Kieselgelsäule chromatographiert. Die Titelverbindung wird als gelbes Oel erhalten, welches beim Stehenlassen erstarrt. Smp. 161-163°.

H.2: Herstellung von 2-(4,4-Dimethyl-4,5-dihydro-1,3-oxazol-2-yl)-1-(3,4-dimethoxyphenyl)-naphthalin (Zwischenprodukt).

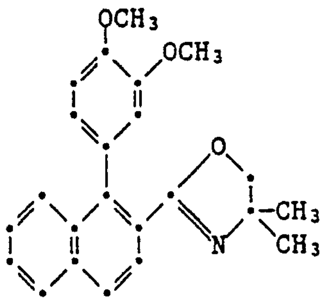

Zu 25,5 g aus 23,2 g 4-Brom-veratrol und 2,5 g Magesium in 50 ml Tetrahydrofuran hergestelltem Grignard-Reagens werden bei 50° 21,3 g 2-(4,4-Dimethyl-4,5-dihydro-1,3-oxazol-2-yl)-1-methoxy-naphthalin zugetropft, die rote Reaktionsmischung zum Sieden erhitzt, 4 h bei Rückfluss gerührt, dann in der Kälte mit ~200 ml gesättigter Ammoniumchlorid-Lösung versetzt und 3 x mit je 100 ml Ether extrahiert. Die Etherphasen werden vereinigt, 2 x mit je 200 ml Wasser gewaschen, über Natriumsulfat getrocknet und das Lösungsmittel unter Vakuum verdampft. Der ölige Rückstand wird zur Reinigung mittels Hexan/Ethylacetat 1:1 über eine Kieselgelsäule chromatographiert. Die Verbindung wird als gelbes Oel erhalten

$n_D^{24.5}$ : 1,6158.

H.3: Herstellung von 1-(3,4-Dimethoxyphenyl)-2-naphthoesäure (Zwischenprodukt).

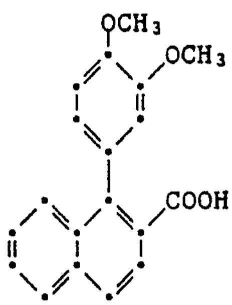

16,0 g des unter H 2 hergestellten 2-(4,4-Dimethyl-4,5-dihydro-1,3-oxazol-2-yl)-1-(3,4-dimethoxyphenyl)-naphthalins werden 8 h in 40 ml 3 N Salzsäure gekocht. Anschliessend wird das zweiphasige Gemisch auf Raumtemperatur abgekühlt und die Phasen werden getrennt. Die schwere Phase wird mit 40 ml 20 %iger methanolischer (Methanol/$H_2O$ 1:1) Natronlauge während 40 Stunden bei Rückfluss gerührt, das Methanol wird abdestilliert, der wässerige Rückstand wird mit 100 ml Wasser verdünnt und 3 x mit je 100 ml Ethylacetat extrahiert. Die wässerige Phase wird mit conc. Salzsäure auf pH 1 gestellt. Die dabei ausgefallenen Kristalle werden abgenutscht, und 2 x mit je 100 ml Wasser gewaschen und im Vakuumtrockenschrank bei 60° C getrocknet. Smp. >215° C.

H.4: Herstellung von 1-(3,4-Dimethoxyphenyl)-6-chlor-2-naphthoesäure morpholid (Verb. Nr. 3.1, Tabelle 3).

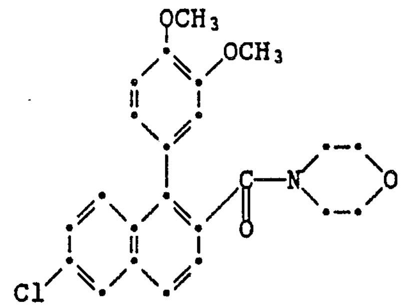

6,9 g 1-(3,4-Dimethoxyphenyl)-6-chlor-2-naphthoesäure werden in 50 ml Toluol vorgelegt und mit 2,4 g Thionylchlorid versetzt. Die Reaktionsmischung wird zum Sieden erhitzt und 5 Stunden unter Rückfluss nachgerührt. Anschliessend wird bei ~50° 4,0 g Morpholin zugetropft, wieder zum Sieden erhitzt und weitere 3 Stunden bei Rückfluss gerührt. Nach dem Abkühlen auf Raumtemperatur wird das Reaktionsgut mit 100 ml Toluol verdünnt, die organische Phase 3 x mit je 250 ml Wasser gewaschen, über Natriumsulfat getrocknet, filtriert und am Vakuum das Lösungsmittel abgedampft. Der ölige Rückstand wird zur Reinigung mittels Ethylacetat über eine Kieselgelsäule chromatographiert. Die Titelverbindung wird in Form beiger Kristalle erhalten. Smp. 157-159°.

H.5: Herstellung von 1-(3,4-Dimethoxyphenyl)-6-chlor-2-naphthoesäure (Zwischenprodukt)

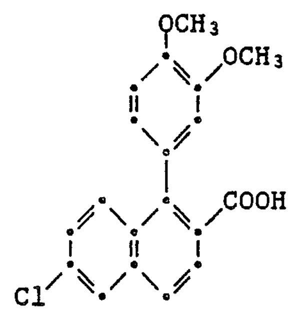

Zu einer Lösung von 20,0 g 1-(3,4-Dimethoxyphenyl)-6-chlor-2-brommagnesiumnaphthalin in 110 ml Tetrahydrofuran, hergestellt aus 18,8 g 1-(3,4-Dimethoxyphenyl)-6-chlor-2-bromnaphthalin und 1,2 g Magnesium werden bei -5 bis 0° trockenes $CO_2$ eingeleitet. Nach Abklingen der exothermen Reaktion wird eine weitere Stunde $CO_2$ eingeleitet. Anschliessend wird unter Kühlen 30 ml ~10 %ige Salzsäure, dann noch 200 ml Wasser zugtropft, die saure Reaktionslösung wird mit konz. Natronlauge alkalisch gestellt und 2 x mit je 150 ml Ether extrahiert. Die wässrige Phase wird mit 20 %iger Salzsäure auf pH 1 gestellt. Die dabei ausgefallene Säure wird abfiltriert, mit wenig Wasser gewaschen und im Vakuumtrockenschrank bei 80° getrocknet. Smp. der beigen Titelverbindung >200°.

H.6: Herstellung von 1-(3,4-Dimethoxyphenyl)-6-chlor-2-bromnaphthalin (Zwischenprodukt).

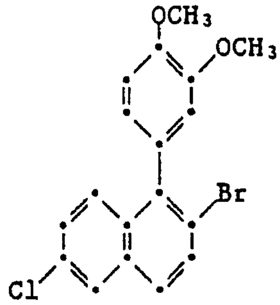

Zu 13,6 g 2,3-Dichlor-5,6-dicyan-1,4-benzochinon in 300 ml Dichlorethan werden 19,0 g 1-(3,4-Dimethoxyphenyl)-6-chlor-2-brom-3,4-dihydronaph thalin, gelöst in 100 ml Dichlorethan, zugetropft, erhitzt und 16 Stunden lang unter Rückfluss gehalten. Das erkaltete Reaktionsgemisch wird anschliessend mit 300 ml Hexan versetzt, filtriert und das Filtrat eingedampft. Der Rückstand wird zur Reinigung mittels Ethylacetat/Hexan 2:1 über eine Kieselgelsäule chromatographiert. Das Produkt wird als weisses Pulver erhalten. Smp. 117-119°.

H.7: Herstellung von 1-(3,4-Dimethoxyphenyl)-6-chlor-2-brom-3,4-dihydronaphthalin (Zwischenprodukt).

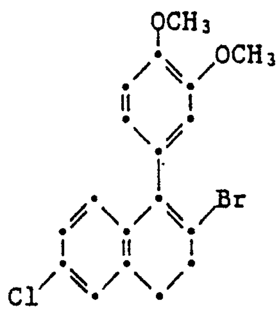

30,0 g 1-(3,4-Dimethoxyphenyl)-6-chlor-3,4-dihydronaphthalin werden in 200 ml Methylenchlorid gelöst und auf 0° abgekühlt. Dazu wird bei 0° bis 5° während 1 1/2 Stunden portionenweise 31,2 g Pyridinhydrobromid-perbromid zugegeben. Nach beendigter Zugabe wird die violette Lösung 2 Stunden bei 0° gerührt, dann mit 300 ml 10 %iger Natriumbicarbonatlösung versetzt, die organische Phase abgetrennt und mit Wasser gewaschen, über Natriumsulfat getrocknet, filtiert und eingedampft. Das so erhaltene, halbkristalline Produkt kann direkt weiter umgesetzt werden. Beim längeren Stehenlassen kristallisiert die Titelverbindung vollständig aus und kann in Hexan digeriert werden. Smp. 92-95°.

H.8: Herstellung von 1-(3,4-Dimethoxyphenyl)-6-chlor-3,4-dihydronaphthalin (Zwischenprodukt).

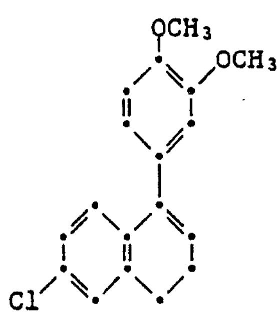

Zu 96,4 g 4-Brommagnesiumveratrol, hergestellt aus 9,6 g Magnesium und 86,8 g 4-Bromveratrol, in 500 ml Tetrahydrofuran werden bei 0 bis max. 5° 43,0 g 6-Chlor-1-tetralon, gelöst in 300 ml Tetrahydrofuran, während 1 Stunde zugetropft. Anschliessed wird das Reaktionsgut eine Stunde lang bei Raumtemperatur und dann 5 Stunden lang bei Rückfluss gerührt, dann auf Raumtemperatur abgekühlt und in der Kälte mit 150 ml 10 %-iger Salzsäure und 500 ml Wasser versetzt. Das Reaktionsgemisch wird 3 x mit je 250 ml Ether extrahiert, die organischen Phasen vereinigt, mit 2 x je 150 ml Wasser gewaschen, über Natriumsulfat getrocknet, filtriert und eingedampft. Der ölige Rückstand wird zur vollständigen Wasserabspaltung während 2 Stunden in 150 ml Methanol/Schwefelsäure (1:1) unter Rückfluss gekocht. Anschliessend wird das Olefin mit 3 x je 250 ml Ether aus der erkalteten Reaktionslösung extrahiert. Die organischen Phasen werden vereinigt, mit Wasser gewaschen, über $Na_2SO_4$ getrocknet, filtriert und eingedampft. Der olige Rückstand wird zur Reinigung mittels Ethylacetet/Hexan 1:2 säulenchromatographiert. Die Titelverbindung wird als gelbes Oel erhalten.

In Analogie zu den Beispielen H.1 und H.4 oder nach einer der weiter oben angegebenen Methoden lassen sich die in den Tabellen 1-3 aufgeführten Verbindungen herstellen.

Tabelle 1

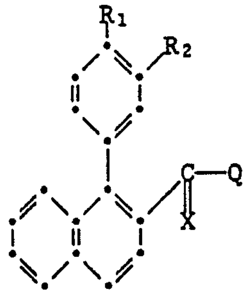

$Q_1$ = 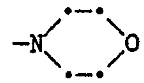

$Q_2$ = 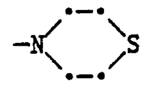

Ph = 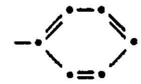

| Verb. Nr. | $R_1$ | $R_2$ | X | Q | Phys. Konst. |
|---|---|---|---|---|---|
| 1.1. | $OCH_3$ | $OCH_3$ | O | $Q_1$ | Smp.161-163° |
| 1.2. | $OCH_3$ | $OCH_3$ | O | $Q_2$ | Smp. 78- 79° |
| 1.3. | $OC_2H_5$ | Cl | O | $Q_1$ | Smp. 76- 81° |
| 1.4. | $OCH_3$ | $CH_3$ | O | $Q_2$ | |
| 1.5. | $OCH_3$ | $OC_2H_5$ | O | $Q_1$ | |
| 1.6. | $OCH_3$ | $OCH_3$ | S | $Q_1$ | Smp. 87- 92° |
| 1.7. | $OCH_3$ | $OCH_3$ | O | $-NHCH(CH_3)_2$ | Smp.181-186° |
| 1.8. | $OCH_3$ | $OCH_3$ | O | 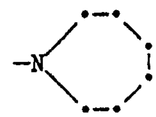 | Smp. 60- 65° |
| 1.9. | $OC_2H_5$ | Cl | O | $Q_2$ | Smp.120-123° |
| 1.10. | $OCH_3$ | Cl | O | $Q_1$ | Smp. 76- 82° |
| 1.11. | $OC_2H_5$ | $OC_2H_5$ | O | $Q_1$ | |
| 1.12. | $OC_2H_5$ | $OCH_3$ | O | $Q_2$ | |
| 1.13. | $OCHF_2$ | $CH_3$ | O | $Q_1$ | |
| 1.14. | $OCH_3$ | $OCH_3$ | O | 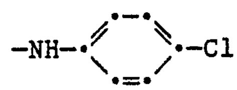 | Smp.190-193° |
| 1.15. | Cl | Cl | O | $Q_1$ | |
| 1.16. | $OCH_3$ | $CH_3$ | O | $Q_1$ | |
| 1.17. | $OCH_3$ | $OCH_3$ | O | 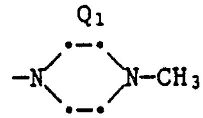 | Smp.108-110° |
| 1.18. | $CH_3$ | $CH_3$ | O | $Q_1$ | |
| 1.19. | $CH_3$ | $OCH_3$ | O | $Q_1$ | |
| 1.20. | $OCH_3$ | Cl | O | $Q_2$ | Smp.150-152° |
| 1.21. | Cl | $CH_3$ | O | $Q_1$ | |
| 1.22. | $OCH_3$ | $OCH_3$ | O | -NH-Ph | Smp.158-163° |
| 1.23. | $OCHF_2$ | $CH_3$ | O | $Q_2$ | |
| 1.24. | $-OCH_2O-$ | | O | $Q_1$ | Smp. 74- 80° |
| 1.25. | $-OCH_2O-$ | | O | -NH-Ph | |
| 1.26. | $-OCH_2O-$ | | O | 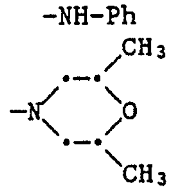 | Smp.118-134° |
| 1.27. | $-OCH_2O-$ | | O | 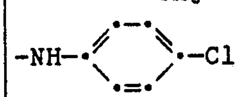 | |

Tab. 1 (Fortsetzung)

| Verb. Nr. | $R_1$ | $R_2$ | X | Q | Phys. Konst. |
|---|---|---|---|---|---|
| 1.28. | $-OCH_2O-$ | | S | $Q_1$ | Smp.168-170° |
| 1.29. | $-OCH_2O-$ | | O | $-N(CH_3)_2$ | |
| 1.30. | $-OCH_2O-$ | | O | $-NHCH(CH_3)_2$ | |
| 1.31. | $-OCH_2O-$ | | O | 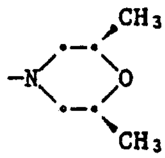 | Smp.144-146° |
| 1.32. | $-OCH_2O-$ | | O | 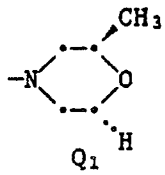 | Smp.103-107° |
| 1.33. | $-OCH_2CH_2O-$ | | O | $Q_1$ | Smp.136-139° |
| 1.34. | $-OCH_2CH_2O-$ | | O | 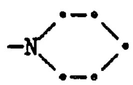 | |
| 1.35. | $-OCH_2CH_2O-$ | | O | $Q_2$ | Smp.127-129° |
| 1.36. | $-OCF_2O-$ | | O | $Q_1$ | |
| 1.37. | $-OCF_2O-$ | | O | $Q_2$ | |
| 1.38. | $-OCH_2O-$ | | S | $Q_2$ | |
| 1.39. | $-OCH_2O-$ | | O | 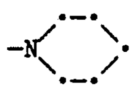 | Smp. 98-100° |
| 1.40. | $-OCH_2O-$ | | O | $Q_2$ | Smp.123-125° |
| 1.41. | $-OCH_2O-$ | | NH | $Q_1$ | |
| 1.42. | $-OCH_2O-$ | | S | $Q_2$ | |
| 1.43. | $OCH_3$ | $OCH_3$ | NH | $Q_1$ | |
| 1.44. | $OCH_3$ | $OCH_3$ | O | $-N(CH_3)_2$ | Smp.147-148° |
| 1.45. | $OCH_3$ | $OCH_3$ | O | 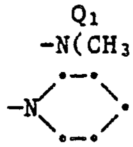 | Smp. 72- 74° |
| 1.46. | $OCH_3$ | $OCH_3$ | O | $-N(C_2H_5)_2$ | Smp. 99-102° |
| 1.47. | $OCHF_2$ | $OCH_3$ | O | $Q_1$ | |
| 1.48. | $CH_3$ | Cl | O | $Q_1$ | |
| 1.49. | $OCHF_2$ | Cl | O | $Q_1$ | |
| 1.50. | $OCH_3$ | $OCH_3$ | O | 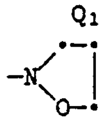 | |
| 1.51. | Cl | Cl | O | $Q_2$ | |
| 1.52. | $N(CH_3)_2$ | Cl | O | $Q_1$ | |
| 1.53. | $NH_2$ | $CH_3$ | O | $Q_1$ | |
| 1.54. | $NH_2$ | Cl | O | $Q_1$ | |
| 1.55. | $OCH_3$ | $OCHF_2$ | O | $Q_1$ | |
| 1.56. | $OCH_3$ | $OCH_3$ | O | $-N(CH_2CH_2OCH_3)_2$ | |
| 1.57. | $OCH_3$ | Cl | O | 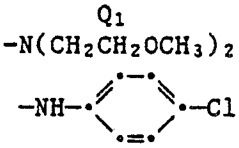 | Smp.202-203° |
| 1.58. | $NH_2$ | $CH_3$ | O | $Q_2$ | |
| 1.59. | Cl | Cl | S | $Q_1$ | |
| 1.60. | $OCHF_2$ | $OCHF_2$ | O | $Q_1$ | |

Tabelle 2

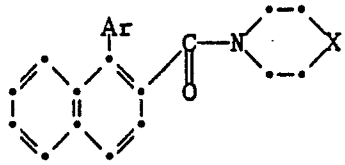

| Verb. Nr. | Ar | X | Physikal. Konstante |
|---|---|---|---|
| 2.1. | 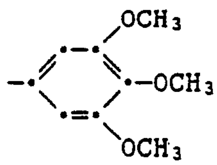 | O | Smp. 118-121° |
| 2.2 | 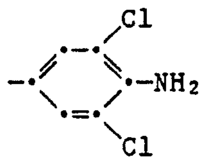 | O | |
| 2.3. | 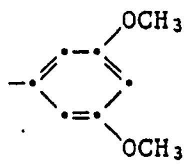 | O | amorph |
| 2.4. | 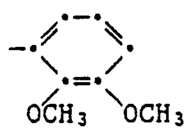 | O | Smp. 132-133° |
| 2.5. | 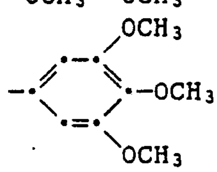 | S | |
| 2.6. | 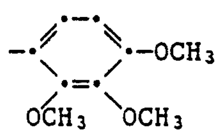 | O | |
| 2.7. | 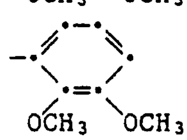 | S | Smp. 140-141° |
| 2.8. | 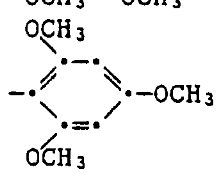 | O | |
| 2.9. | 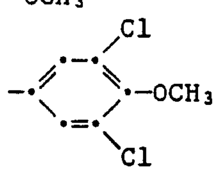 | O | |

Tabelle 3:

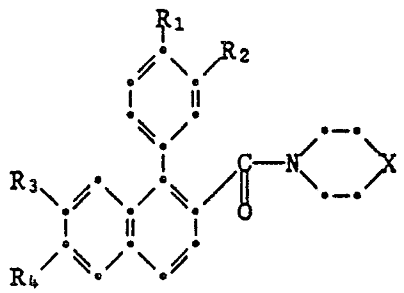

| Verb. Nr. | $R_1$ | $R_2$ | $R_3$ | $R_4$ | X | Physikal. Konstante |
|---|---|---|---|---|---|---|
| 3.1. | $OCH_3$ | $OCH_3$ | H | Cl | O | Smp. 157-159° |
| 3.2. | $OCH_3$ | $OCH_3$ | $OCH_3$ | $OCH_3$ | O | |
| 3.3. | $OCH_3$ | $OCH_3$ | H | $NH_2$ | O | |
| 3.4. | $OCH_3$ | $OCH_3$ | H | Cl | S | |
| 3.5. | $OCH_3$ | $OCH_3$ | H | $NH-COCH_3$ | O | |
| 3.6. | $OCH_3$ | $OCH_3$ | $-OCH_2O-$ | | O | |
| 3.7. | $OCH_3$ | $OCH_3$ | $-OCH_2O-$ | | S | |
| 3.8. | $OCH_3$ | $OCH_3$ | H | Cl | $CH_2$ | |
| 3.9. | $OCH_3$ | $OCH_3$ | H | Cl | $N-CH_3$ | |
| 3.10. | $OC_2H_5$ | Cl | H | Cl | O | |
| 3.11. | $OCH_3$ | $OC_2H_5$ | H | Cl | O | |
| 3.12. | Cl | Cl | H | Cl | O | |
| 3.13. | $OCHF_2$ | $OCHF_2$ | H | Cl | O | |
| 3.14. | $NH_2$ | $CH_3$ | H | Cl | O | |
| 3.15. | $-OCH_2O-$ | | H | Cl | O | |
| 3.16. | $-OCH_2O-$ | | $-OCH_2O-$ | | O | |
| 3.17. | $-OCH_2O-$ | | H | Cl | S | |
| 3.18. | $-OCH_2O-$ | | $OCH_3$ | $OCH_3$ | O | |
| 3.19. | $-OCH_2O-$ | | H | Cl | S | |
| 3.20. | $-OCH_2O-$ | | H | Cl | $CH_2$ | |
| 3.21. | $-OCH_2O-$ | | H | $NH_2$ | O | |
| 3.22. | $-OCH_2O-$ | | H | $NHCOCH_3$ | O | |
| 3.23. | $-OCH_2O-$ | | H | Cl | $N-CH_3$ | |
| 3.24. | $OCH_3$ | $OCH_3$ | H | $OCH_3$ | O | |
| 3.25. | $OCH_3$ | $OC_2H_5$ | H | $OCH_3$ | O | |
| 3.26. | $OCH_3$ | $OCH_3$ | H | $OCH_3$ | S | |
| 3.27. | $OCH_3$ | $OCH_3$ | $-OCF_2O-$ | | O | |
| 3.28. | $-OCF_2O-$ | | H | Cl | O | |

## 2. Formulierungsbeispiele für Wirkstoffe der Formel I (% = Gewichtsprozent)

### 2.1. Emulsion-Konzentrate

| | a) | b) | c) |
|---|---|---|---|
| Wirkstoff aus den Tabellen 1 bis 3 | 25% | 40% | 50% |
| Ca-Dodecylbenzolsulfonat | 5% | 8% | 6% |
| Rizinusöl-polyäthylenglykoläther (36 Mol Aethylenoxid) | 5% | - | - |
| Tributylphenoyl-polyäthylenglykoläther (30 Mol Aethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

2.2. Lösungen

| | a) | b) | c) | d) |
|---|---|---|---|---|
| Wirkstoff aus den Tabellen 1 bis 3 | 80% | 10% | 5% | 95% |
| Aethylenglykol-monomethyläther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190° C) | - | - | 94% | - |
| (MG = Molekulargewicht) | | | | |

Diese Lösungen eignen sich für die Applikation als Mikrodispersionen.

2.3. Granulate

| | a) | b) |
|---|---|---|
| Wirkstoff aus den Tabellen 1 bis 3 | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

2.4. Stäubemittel

| | a) | b) |
|---|---|---|
| Wirkstoff aus den Tabellen 1 bis 3 | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

2.5. Spritzpulver

| | a) | b) | c) |
|---|---|---|---|
| Wirkstoff aus den Tabellen 1 bis 3 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol Aethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

3. Biologische Beispiele

Beispiel 3.1: Wirkung gegen Plasmopara viticola auf Reben

a) Residual-protektive Wirkung

Im 4-5 Blattstadium wurden Rebensämlinge mit einer aus Spritzpulver des Wirkstoffes hergestellten Spritzbrühe (0,02 % Aktivsubstanz) besprüht. Nach 24 Stunden wurden die behandelten Pflanzen mit einer Sporangiensuspension des Pilzes infiziert. Nach einer Inkubation während 6 Tagen bei 95-100 % relativer Luftfeuchtigkeit und 20° C wurde der Pilzbefall beurteilt.

b) Residual-kurative Wirkung

Im 4-5 Blattstadium wurden Rebensämlinge mit einer Sporangiensuspension des Pilzes infiziert Nach einer Inkubation während 24 Stunden in einer Feuchtkammer bei 95-100 % relativer Luftfeuchtigkeit und 20° C wurden die infizierten Pflanzen getrocknet und mit einer aus Spritzpulver des Wirkstoffes hergestellten Spritzbrühe (0,02 % Aktivsubstanz) besprüht. Nach dem Antrocknen des Spritzbelages wurden die behandelten Pflanzen wieder in die Feuchtkammer gebracht. Die Beurteilung des Pilzbefalls erfolgte 6 Tage nach der Infektion.

Verbindungen aus Tabelle 1 bis 3 zeigten gegen Plasmopara viticola auf Reben eine sehr gute fungizide Wirkung, insbesondere die Wirkstoffe Nr. 1.1, 1.2, 1.6, 1.8, 1.24, 1.46 und 3.1 bewirkten eine vollständige Unterdrückung des Pilzbefalls (Restbefall 0 bis 5 %).

Beispiel 3.2: Wirkung gegen Cercospora arachidicola auf Erdnusspflanzen

Residual-protektive Wirkung

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21° C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %) zeigen Erdnusspflanzen, die mit Wirkstoffen aus den Tabellen behandelte werden, einen stark reduzierten Cercospora-Befall. Die Verbindungen 1.24 und 1.26 und andere der Tabellen 1 bis 3 verhinderten das Auftreten von Flecken fast vollständig (0-10 %).

Beispiel 3.3: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24 °C aufgestellt. Der Schorfbefall wird 15 Tage nach der Infektion beurteilt. Die Verbindung Nr. 1.24 der Tabelle 1 hemmt den Krankheitsbefall auf weniger als 10 %. Unbehandelte aber infizierte Kontrolltriebe werden dagegen 100%ig befallen.

Beispiel 3.4.: Wirkung gegen Erysiphe graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konodoen des Pilzes bestäubt. Die infizierten Gerstenpflanzen werden in einem Gewächshaus bei ca. 22 °C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,02 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden werden die behandelten Pflanzen mit Konidien des Pilzes bestäubt. Die infizierten Gerstenpflanzen werden in einem Gewächshaus bei ca. 22 °C aufgestellt und der Pilzbefall nach 10 Tagen beurteilt.

Verbindungen aus den Tabellen 1 bis 3 zeigten gegen Erysiphe gute Wirksamkeit. So reduzieren z.B. die Verbindungen Nr. 1.1, 1.2, 1.10, 1.24, 1.26, 1.31, 1.32, 1.40, 2.1 und 3.1 den Erysiphae-Befall auf 0 bis 5 %. Unbehandelte aber infizierte Kontrollpflanzen weisen dagegen einen Erysiphe-Befall von 100 % auf.

Beispiel 3.5: Wirkung gegen Phytophthora auf Tomatenpflanzen Residualprotektive Wirkung

Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20 °C.

Verbindungen aus den Tabellen 1 bis 3 zeigten eine nachhaltige Wirkung (weniger als 20 % Pilzbefall). Mit den Verbindungen Nr. 1.1, 1.6, 1.46 sowie 3.1, 3.4 und anderen Präparaten der Tabelle 3 wurde ein Befall praktisch vollständig verhindert (0 bis 5 % Befall).

## Ansprüche

1. 1-Aryl-2-naphthoylamine der Formel I

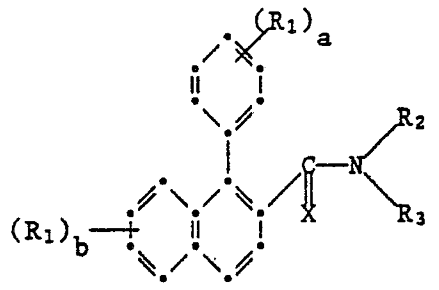

(I)

worin $R_1$, Halogen, Nitro, Cyano, unsubstituiertes oder durch Halogen und/oder $C_1$-$C_3$-Alkoxy ein- oder mehrfach substituiertes $C_1$-$C_6$-Alkyl, $C_3$-$C_6$-Cycloalkyl, $OR_4$, $NR_5R_6$, $CO_2R_5$, $CONR_5R_6$ oder $NHCOR_7$ bedeutet, oder worin zwei benachbarte Positionen im Kern durch eine unsubstituierte oder durch Fluor ein- oder mehrfach substituierte Methylendioxy- oder Aethylendioxy-Gruppe überbrückt wird, worin ferner $R_4$ Wasserstoff oder $C_1$-$C_6$-Alkyl bedeutet, das unsubstituiert oder durch $C_1$-$C_3$-Alkoxy oder ein- oder mehrfach durch Halogen substituiert ist, oder aber für $C_3$-$C_4$-Alkenyl, 2-Propinyl, 3-Halogen-2-propinyl, $-O\underset{\overset{\|}{O}}{C}ZR_7$ oder $COR_7$ steht,

$R_5$ und $R_6$ unabhängig voneinander H oder $C_1$-$C_4$-Alkyl ist,
$R_7$ für $C_1$-$C_4$-Alkyl und
Z für O oder NH steht,
a und b die Zahl der besetzten Positionen im jeweiligen Ring darstellen und
a für die Zahlen 1-3, b für die Zahlen 0-3 stehen, wobei die einzelnen Gruppen $R_1$ gleich oder verschieden sind, wenn die Summe von a und b grösser als 1 ist,
X für O, S oder NH stehen,
$R_2$ und $R_3$ unabhängig voneinander unsubstituiertes oder durch $C_1$-$C_4$-Alkoxy, Halogen, Cyano substituiertes $C_1$-$C_6$-Alkyl oder aber $C_3$-$C_7$-Cycloalkyl, $C_3$-$C_7$-Alkenyl oder den Rest

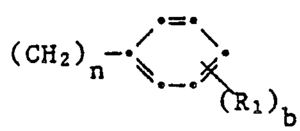

darstellt, worin
n = 0 oder 1 ist, ferner
$R_2$ und $R_3$ auch gemeinsam eine $C_4$-$C_7$-Alkylenkette, welche mit dem sie verbindenden Stickstoffatom einen unsubstituierten oder durch $C_1$-$C_4$-Alkyl mono- oder disubstituierten Heterocyclus bilden kann, wobei eine Methylengruppe aus dieser Kette durch O, S oder $NR_7$ ersetzt sein kann, sowie $R_2$ auch Wasserstoff bedeuten kann; einschliesslich der Additionssalze der Verbindungen der Formel I.

2. 1-Aryl-2-naphthoylamine gemäss Anspruch 1, worin $R_1$ Halogen, Nitro, $C_1$-$C_6$-Alkyl, $C_3$-$C_6$-Cycloalkyl, Methylendioxy, Mono- oder Difluormethylendioxy, unsubstituiertes oder durch Fluor ein- oder mehrfach substituiertes Aethylendioxy, $OR_4$ oder $NR_5R_6$ bedeuten, worin
$R_4$ Wasserstoff oder $C_1$-$C_3$-Alkyl darstellt, das unsubstituiert oder durch $C_1$-$C_3$-Alkoxy monosubstituiert oder durch Halogen ein- bis fünffach substituiert ist,
$R_5$ und $R_6$ unabhängig voneinander für H oder $C_1$-$C_4$-Alkyl,
a für die Zahlen 2 oder 3, b für 0 oder 1, und
X für O oder S stehen, während
$R_2$ und $R_3$ unabhängig voneinander Phenyl oder 4-Chlorphenyl sind, ferner
$R_2$ und $R_3$ auch gemeinsam eine $C_4$-$C_7$-Alkylenkette, welche zusammen mit dem Stickstoffatom einen unsubstituierten oder durch $C_1$-$C_4$-Alkyl mono- oder disubstituierten Heterocyclus bilden können, wobei eine der Methylengruppen aus der Kette durch O, S oder $NR_7$ ersetzt sein kann.

3. 1-Aryl-2-naphthoylamine gemäss Anspruch 2 worin $R_1$ Methyl, Chlor, Methoxy, Aethoxy, Difluormethoxy, Methylendioxy, Difluormethylendioxy, Aethylendioxy, Nitro, Mono- oder Dimethylamino bedeutet und $R_2$ und $R_3$ mit dem gemeinsamen Stickstoffatom zusammen die Reste

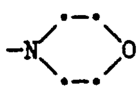 , 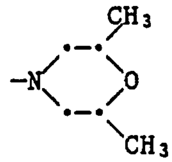 oder 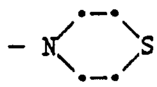

bilden.

4. 1-Aryl-2-naphthoylamine gemäss Anspruch 3, worin $R_1$ Methoxy oder Methylendioxy bedeutet und a für die Zahl 2 steht.

5. 1-Aryl-2-naphthoylamine gemäss Anspruch 4, worin X für O steht.

6. Die Verbindung der Formel Ia

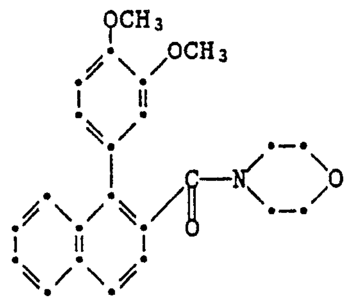

(Ia)

gemäss Anspruch 5.

7. 1-Aryl-2-naphthoylamine gemäss Anspruch 1, worin $R_1$ Methoxy oder Methyl bedeuten, a für die Zahlen 2 oder 3 und b für die Zahl 0 steht, während X Schwefel bedeutet.

8. Die Verbindung der Formel Ib

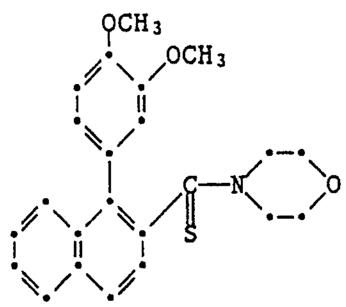

(Ib)

gemäss Anspruch 7.

9. 1-Aryl-2-naphthoylamine gemäss Anspruch 1, bei welchen $R_1$ Halogen, Nitro, $C_1$-$C_6$-Alkyl, $C_1$-$C_3$-Alkoxy, $C_3$-$C_6$-Cycloalkyl, Methylendioxy, Monooder Difluormethylendioxy, unsubstituiertes oder durch Fluor ein- oder mehrfach substituiertes Aethylendioxy oder $NR_5R_6$ bedeuten, worin
$R_5$ und $R_6$ unabhängig voneinander für H oder $C_1$-$C_4$-Alkyl,
a für die Zahlen 2 oder 3 und b für 0 oder 1, und
X für O oder S stehen, und
$R_2$ und $R_3$ unabhängig voneinander für Wasserstoff, unsubstituiertes oder durch $C_1$-$C_4$-Alkoxy, Halogen oder Cyano substituiertes $C_1$-$C_6$-Alkyl, oder oder $C_3$-$C_7$-Alkenyl, sowie Phenyl oder 4-Chlorphenyl stehen, ferner $R_2$ und $R_3$ auch gemeinsam eine $C_4$-$C_7$-Alkylenkette darstellt, welche mit dem Stickstoffatom einen unsubstituierten oder durch $C_1$-$C_4$-Alkyl mono- oder disubstituierten Heterocyclus bilden können, wobei eine der Methylengruppen aus der Kette durch O, S oder $NR_7$ ersetzt sein kann, und die der Formel Ic gehorchen

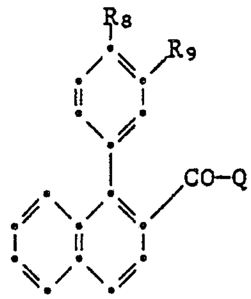

(Ic),

worin $R_8$ und $R_9$ entweder jeweils den Methoxyrest oder zusammen den Methylendioxyrest und Q die folgenden Reste bedeuten:

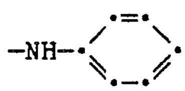 , 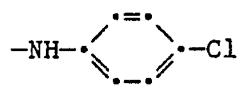 ,

$-N(CH_3)_2$ , $-N(C_2H_5)_2$ , $-N(C_2H_4OCH_3)_2$ ,

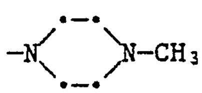 , 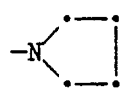 , 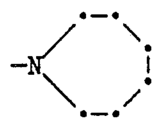 , 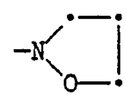 .

10. Die Verbindung der Formel Id

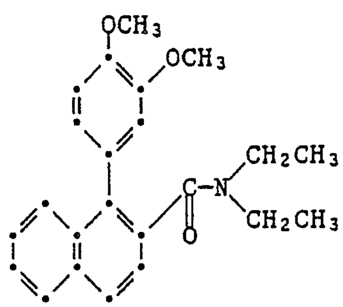

(Id),

gemäss Anspruch 9.

11. 1-Aryl-2-naphthoylamine gemäss Anspruch 1 der Formel Ie

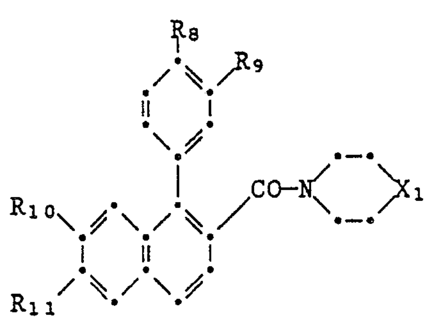

(Ie),

worin $R_8$ und $R_9$ beide entweder Methoxy oder zusammen Methylendioxy bedeuten,
$R_{10}$ und $R_{11}$ Methoxy oder zusammen Methylendioxy bedeuten und
$R_{11}$ ausserdem Chlor, Nitro, Amino, Acetylamino sein kann, während

$R_{10}$ Wasserstoff ist und
$X_1$ O oder S bedeuten.

12. Verfahren zur Herstellung der Verbindungen der Formel I gemäss Anspruch 1, dadurch gekennzeichnet, dass man ein Amin der Formel II

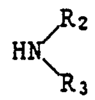

(II),

worin $R_2$ und $R_3$ die im Anspruch 1 angegebenen Bedeutungen haben,

a) mit einem Naphthoesäurederivat der Formel III

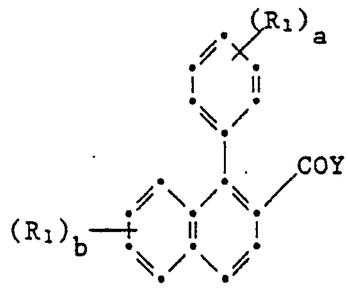

(III),

worin $R_1$, a und b die im Anspruch 1 angegebenen Bedeutungen haben und Y OH, Halogen oder $C_1$-$C_4$-Alkoxy bedeutet, in eine Verbindung der Formel I′

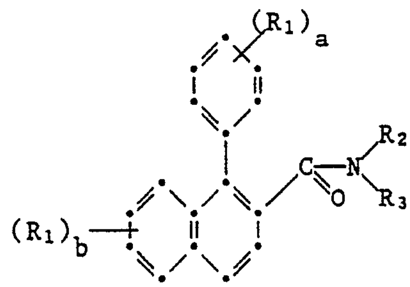

(I′)

überführt;und, sofern gewünscht
die Verbindung I′ mit Phosphorpentasulfid in ein Thiocarbonamid der Formel I″

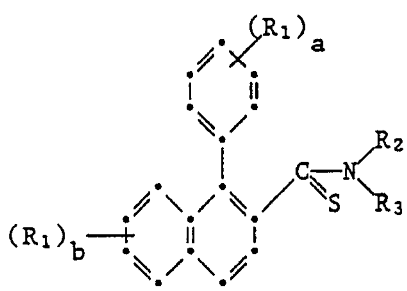

(I″)

verwandelt;
oder zur Erzielung von Verbindungen der Formel I mit X = NH,

b) mit einem Iminoester der Formel IV

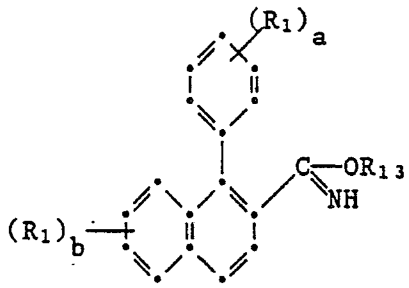

(IV)

worin $R_1$, a und b die im Anspruch 1 angegebenen Bedeutungen haben und $R_{13}$ $C_1$-$C_4$-Alkyl bedeutet, in eine Verbindung der Formel I'''

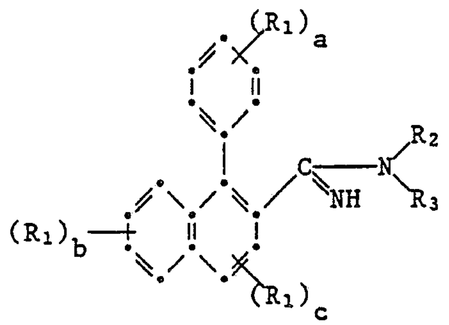

(I''')

überführt.

13. Mikrobizides Mittel enthaltend neben den üblichen Trägersubstanzen und Zuschlagstoffen mindestens eine der Verbindungen der Formel I gemäss Anspruch 1 als aktive Komponente.

14. Mittel nach Anspruch 13, dadurch gekennzeichnet, dass es als aktive Komponente die Verbindung der Formel Ia gemäss Anspruch 6 enthält.

15. Mittel nach Anspruch 13, dadurch gekennzeichnet, dass es als aktive Komponente die Verbindung der Formel Ib gemäss Anspruch 8 enthält.

16. Mittel nach Anspruch 13, dadurch gekennzeichnet, dass es als aktive Komponente die Verbindung der Formel Id gemäss Anspruch 10 enthält.

17. Verfahren zur Bekämpfung pflanzenschädigender Mikroben, dadurch gekennzeichnet, dass man auf die Pflanze oder deren Standort eine mikrobizid wirksame Menge einer Verbindung der Formel I gemäss Anspruch 1 appliziert.

18. Verfahren nach Anspruch 17, dadurch gekennzeichnet, dass die Mikroben Plasmopara, Phytophtora Venturia, Cercospora oder Erysiphe sind.

Patentansprüche für folgenden Vertragsstaat:ES

1. Verfahren zur Herstellung der Verbindungen der Formel I

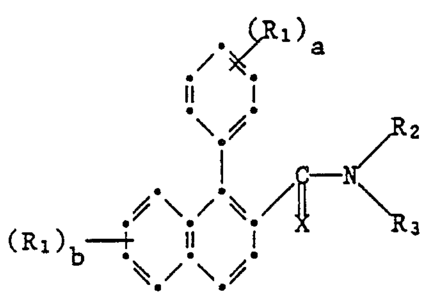

(I)

worin $R_1$, Halogen, Nitro, Cyano, unsubstituiertes oder durch Halogen und/oder $C_1$-$C_3$-Alkoxy ein- oder

mehrfach substituiertes $C_1$-$C_6$-Alkyl, $C_3$-$C_6$-Cycloalkyl, $OR_4$, $NR_5R_6$, $CO_2R_5$, $CONR_5R_6$ oder $NHCOR_7$ bedeutet, oder worin zwei benachbarte Positionen im Kern durch eine unsubstituierte oder durch Fluor ein- oder mehrfach substituierte Methylendioxy- oder Aethylendioxy-Gruppe überbrückt wird, worin ferner $R_4$ Wasserstoff oder $C_1$-$C_6$-Alkyl bedeutet, das unsubstituiert oder durch $C_1$-$C_3$-Alkoxy oder ein- oder mehrfach durch Halogen substituiert ist, oder aber für $C_3$-$C_4$-Alkenyl, 2-Propinyl, 3-Halogen-2-propinyl, $-O\underset{\underset{O}{\|}}{C}ZR_7$

oder $COR_7$ steht,
$R_5$ und $R_6$ unabhängig voneinander H oder $C_1$-$C_4$-Alkyl ist,
$R_7$ für $C_1$-$C_4$-Alkyl und
Z für O oder NH steht,
a und b die Zahl der besetzten Positionen im jeweiligen Ring darstellen und
a für die Zahlen 1-3, b für die Zahlen 0-3 stehen, wobei die einzelnen Gruppen $R_1$ gleich oder verschieden sind, wenn die Summe von a und b grösser als 1 ist,
X für O, S oder NH stehen,
$R_2$ und $R_3$ unabhängig voneinander unsubstituiertes oder durch $C_1$-$C_4$-Alkoxy, Halogen, Cyano substituiertes $C_1$-$C_6$-Alkyl oder aber $C_3$-$C_7$-Cycloalkyl, $C_3$-$C_7$-Alkenyl oder den Rest

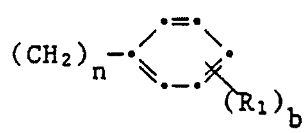

darstellt, worin
n = 0 oder 1 ist, ferner
$R_2$ und $R_3$ auch gemeinsam eine $C_4$-$C_7$-Alkylenkette, welche mit dem sie verbindenden Stickstoffatom einen unsubstituierten oder durch $C_1$-$C_4$-Alkyl mono- oder disubstituierten Heterocyclus bilden kann, wobei eine Methylengruppe aus dieser Kette durch O, S oder $NR_7$ ersetzt sein kann, sowie $R_2$ auch Wasserstoff bedeuten kann, dadurch gekennzeichnet, dass man ein Amin der Formel II

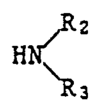 (II),

worin $R_2$ und $R_3$ die im Anspruch 1 angegebenen Bedeutungen haben,
a) mit einem Naphthoesäurederivat der Formel III

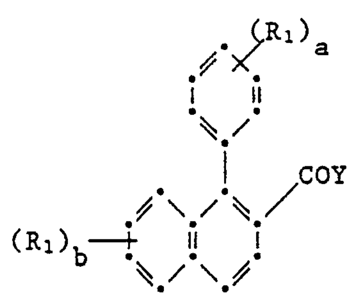 (III),

worin $R_1$, a und b die im Anspruch 1 angegebenen Bedeutungen haben und Y OH, Halogen oder $C_1$-$C_4$-Alkoxy bedeutet, in eine Verbindung der Formel I'

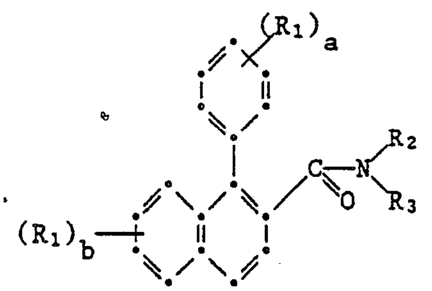

(I')

überführt;und, sofern gewünscht die Verbindung I′ mit Phosphorpentasulfid in ein Thiocarbonamid der Formel I″

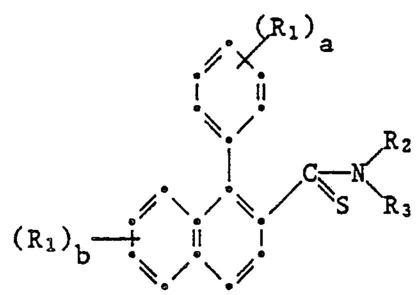

(I'')

verwandelt;
oder zur Erzielung von Verbindungen der Formel I mit X = NH,
b) mit einem Iminoester der Formel IV

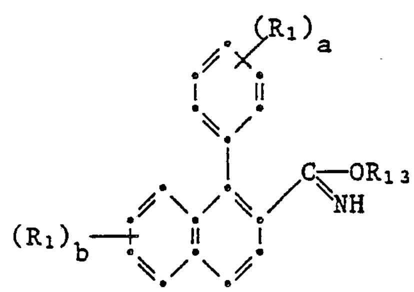

(IV)

worin $R_1$, a und b die im Anspruch 1 angegebenen Bedeutungen haben und $R_{13}$ $C_1$-$C_4$-Alkyl bedeutet, in eine Verbindung der Formel I‴

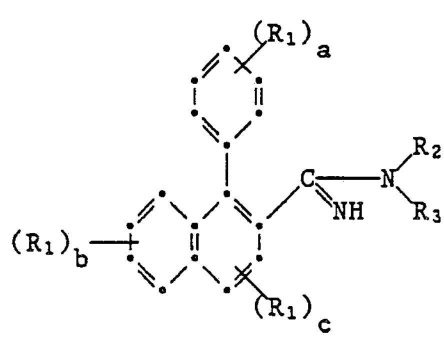

(I''')

überführt.

2. Verfahren gemäss Anspruch 1, dadurch gekennzeichnet, dass $R_1$ Halogen, Nitro, $C_1$-$C_6$-Alkyl, $C_3$-$C_6$-Cycloalkyl, Methylendioxy, Mono- oder Difluormethylendioxy, unsubstituiertes oder durch Fluor ein- oder mehrfach substituiertes Aethylendioxy, $OR_4$ oder $NR_5R_6$ bedeuten, worin
$R_4$ Wasserstoff oder $C_1$-$C_3$-Alkyl darstellt, das unsubstituiert oder durch $C_1$-$C_3$-Alkoxy monosubstituiert oder durch Halogen ein- bis fünffach substituiert ist,
$R_5$ und $R_6$ unabhängig voneinander für H oder $C_1$-$C_4$-Alkyl,
a für die Zahlen 2 oder 3, b für 0 oder 1, und
X für O oder S stehen, während
$R_2$ und $R_3$ unabhängig voneinander Phenyl oder 4-Chlorphenyl sind, ferner
$R_2$ und $R_3$ auch gemeinsam eine $C_4$-$C_7$-Alkylenkette, welche zusammen mit dem Stickstoffatom einen unsubstituierten oder durch $C_1$-$C_4$-Alkyl mono- oder disubstituierten Heterocyclus bilden können, wobei eine der Methylengruppen aus der Kette durch O, S oder $NR_7$ ersetzt sein kann.

3. Verfahren gemäss Anspruch 2, dadurch gekennzeichnet, dass $R_1$ Methyl, Chlor, Methoxy, Aethoxy, Difluormethoxy, Methylendioxy, Difluormethylendioxy, Aethylendioxy, Nitro, Mono- oder Dimethylamino bedeutet und $R_2$ und $R_3$ mit dem gemeinsamen Stickstoffatom zusammen die Reste

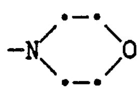 , 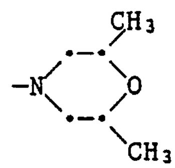 oder 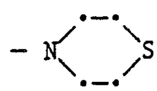

bilden.

4. Verfahren gemäss Anspruch 3, dadurch gekennzeichnet, dass $R_1$ Methoxy oder Methylendioxy bedeutet und a für die Zahl 2 steht.

5. Verfahren gemäss Anspruch 4, dadurch gekennzeichnet, dass X für O steht.

6. Verfahren gemäss Anspruch 5 zur Herstellung der Verbindung der Formel Ia

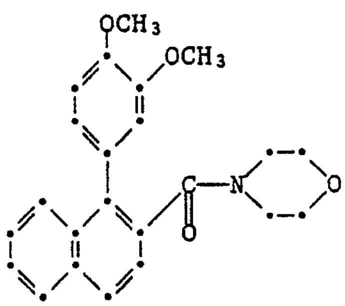 (Ia)

7. Verfahren gemäss Anspruch 1, dadurch gekennzeichnet, dass $R_1$ Methoxy oder Methyl bedeuten, a für die Zahlen 2 oder 3 und b für die Zahl 0 steht, während X Schwefel bedeutet.

8. Verfahren gemäss Anspruch 7 zur Herstellung der Verbindung der Formel Ib

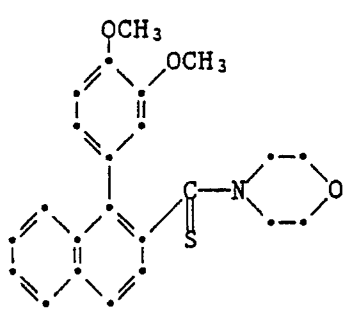 (Ib)

9. Verfahren gemäss Anspruch 1, dadurch gekennzeichnet, dass $R_1$ Halogen, Nitro, $C_1$-$C_6$-Alkyl, $C_1$-$C_3$-Alkoxy, $C_3$-$C_6$-Cycloalkyl, Methylendioxy, Mono-oder Difluormethylendioxy, unsubstituiertes oder durch Fluor einoder mehrfach substituiertes Aethylendioxy oder $NR_5R_6$ bedeuten, worin
$R_5$ und $R_6$ unabhängig voneinander für H oder $C_1$-$C_4$-Alkyl,
a für die Zahlen 2 oder 3 und b für 0 oder 1, und
X für O oder S stehen, und
$R_2$ und $R_3$ unabhängig voneinander für Wasserstoff, unsubstituiertes oder durch $C_1$-$C_4$-Alkoxy, Halogen oder Cyano substituiertes $C_1$-$C_6$-Alkyl, oder oder $C_3$-$C_7$-Alkenyl, sowie Phenyl oder 4-Chlorphenyl stehen, ferner $R_2$ und $R_3$ auch gemeinsam eine $C_4$-$C_7$-Alkylenkette darstellt, welche mit dem Stickstoffatom einen unsubstituierten oder durch $C_1$-$C_4$-Alkyl mono- oder disubstituierten Heterocyclus bilden können, wobei eine der Methylengruppen aus der Kette durch O, S oder $NR_7$ ersetzt sein kann, und die der Formel Ic gehorchen

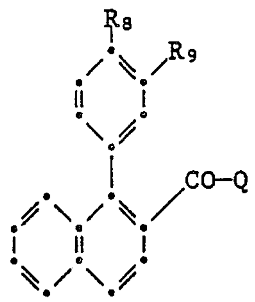

(Ic),

worin $R_8$ und $R_9$ entweder jeweils den Methoxyrest oder zusammen den Methylendioxyrest und Q die folgenden Reste bedeuten:

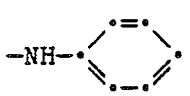 , 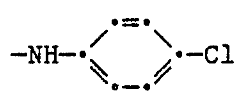 ,

$-N(CH_3)_2$ , $-N(C_2H_5)_2$ , $-N(C_2H_4OCH_3)_2$ ,

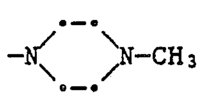 , 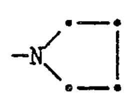 , 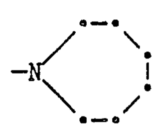 , 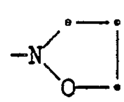 .

10. Verfahren gemäss Anspruch 9 zur Herstellung der Verbindung der Formel Id

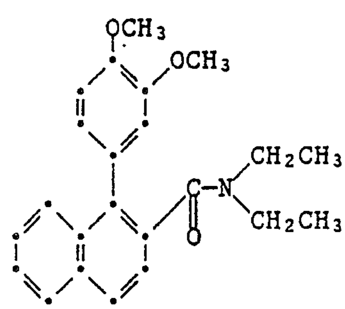

(Id),

11. Verfahren gemäss Anspruch 1 zur Herstellung der 1-Aryl-2-naphthoylamine der Formel Ie